# EUROPEAN PATENT SPECIFICATION

(11) **EP 1 532 949 B1**
(45) Date of publication and mention of the grant of the patent: **18.07.2007**
(21) Application number: 03026820.5
(22) Date of filing: 20.11.2003
(51) Int. Cl.: A61F 2/44

(54) **Expandable intervertebral fusion cage**
Expandierbarer Zwischenwirbelfusionskäfig
Cage de fusion intervertébrale expansible

(43) Date of publication of application: 25.05.2005
(73) Proprietor: Kyungwon Medical Co., Ltd., Seoul 135-924 (KR)
(72) Inventor: Chae, Soo-kyung, 112-204, Kaepo Woosung 7cha Apt., Seoul 135-230 (KR); Youn, Jae-ryoun, Seoul 135-917 (KR); Yoon, Do-heum, 1-908, Ssangyong Apartment, Seoul 135-776 (KR); Cho, Yong-eun, 601-203, Cheonggu Apartment, Seongnam-si Kyeonggi-do 463-905 (KR); Kim, Sang-taek, 3-1006, Samik Apartment, Seoul 137-070 (KR); Park, Hyung-sang, Seoul 138-160 (KR)
(74) Representative: Kühn, Armin

(56) References cited:
- EP-A- 1 290 985
- US-A- 5 980 522
- US-A- 6 117 174
- US-A- 6 129 763

## Description

### BACKGROUND OF THE INVENTION

### Field of the Invention

The present invention relates to an expandable interfusion cage according to the preamble of claim 1.

The present invention relates, in general, to an artificial interfusion implant installed in an intervertebral space created from the removal of a damaged spinal disk, and more particularly, to an expandable interfusion cage, expandable to increase in diameter.

### Description of the Prior Art

In order to stabilize, for extended periods, fractures of a damaged spinal disk in humans, a fusion can be implemented, in which at least two bones of the spinal column are connected with each other by a bony bridge. In implementing interbody fusion as well known to those skilled in the art, so as to restore a normal spatial relationship, a spinal disk is partially cut, and a bone segment is positioned between adjacent vertebrae in a space already occupied by a disk material, to provide immediate stability through mechanical support. Also, by subsequent permanent cross-conjugation of vertebral bones, long-term stability is provided.

The main application of the invention is to provide implants designed to be slid or inserted from a posterior direction between the confronting faces of two consecutive vertebrae in order to maintain a predetermined distance between them and to restore stability to the spinal column, e.g. after a failure of the corresponding joint, by fixing the two vertebrae together.

Several techniques are known at present for restoring a normal lumbar lordosis in this way, by implanting either a graft which in time fuses the vertebrae together, or a prosthesis which fixes them together immediately, while maintaining the possibility of achieving a fusion between the vertebrae in time.

In the second above-mentioned technique, use is made mainly of implants, also known as "cages", some of which are hollow, rigid, and contain only one piece, with inside/outside intercommunication slots for receiving a bone graft which, via said slots, subsequently fuses with the adjacent vertebrae on either side. In this field, reference can be made to International Patent Publication No. WO 96/08205 published on March 21, 1996 for an "Intervertebral fusion cage of conical shape", and European Patent Publication No. EP 637 440 published on February 8, 1995 for an "Intersomatic implant for the spinal column". Nevertheless, cages of those types are of outside dimensions that are given and fixed, whereas the distances between pairs of vertebrae are not constant. In addition, the inclinations of the facing vertebral faces to which a given angular position is to be imparted do not enable rigid cages to be used from a posterior direction. That is, they can be inserted only from an anterior direction.

As a result, other types of cages have been developed with two substantially parallel branches connected to a rigid body through which it is possible to turn a worm screw system which then moves a wedge in a screw engagement on a worm screw from an initial position close to the distal ends of the branches towards the body linking the branches together, thereby splaying the two branches apart angularly. It is then possible to insert such a cage, in an initially flat shape, between the vertebrae, and then by turning the drive axis of the wedge, the desired angle between the branches is adjusted or set from a posterior access.

Such cages or implants are described, for example, in European Patent Publication No. EP 664 994 published on August 2, 1995, entitled "Vertebral intersomatic cage" or in France Patent Publication No. 2 719 763 published on November 17, 1995, entitled "Vertebral implant".

However, such devices which are more mechanical than hollow and rigid cages, and therefore more complex, leave a smaller inside volume for the fusion graft, and because of their flat shape which is not circularly symmetrical, even though they are better at ensuring a given bearing angle between the vertebrae, they require a passage of the same rectangular section to be prepared to receive them, which complicates implementation.

The problem posed is thus to be able to have implants or cages available making it possible simultaneously to ally the shape of a conventional rigid cage, firstly to facilitate implantation and secondly to provide a larger inside volume, with the possibility of increasing the diameter of the distal end of the cage to a given value relative to its end situated adjacent to its point of surgical insertion, after it has been put into place, and corresponding to the posterior face of the vertebrae, while having as few mechanical elements as possible.

In an effort to address this problem, an expandable osteosynthesis cage is disclosed in International Publication No. WO 1998/10722 dated March 19, 1998 and in US patent application no. 6,129,763. In this regard, referring to Figs. 1 and 2, the cage 1 has a hollow shape and includes a seat 7 and branches 5. The seat 7 serves as a cylindrical body and is pierced by an orifice 8. Each of the branches 5 is connected at one end thereof to the seat 7. A spacer 2 is movably assembled in an inside volume 9, that is, an inside space, of the cage 1. As the spacer 2 is moved toward the distal ends of the branches 5, the branches 5 are biased radially outward by the spacer 2 and thereby expanded.

The expandable osteosynthesis cage partially solves the defects occurring in the conventional art, but some of the defects still remain. In particular, while the spacer 2 is movably assembled in the inside space having a tapered section, if the spacer 2 is moved to the distal ends of the branches 5 to bias radially outward the branches 5, positioning of the spacer 2 at the distal ends of the branches 5 is structurally unstable.

### SUMMARY OF THE INVENTION

Accordingly, the present invention has been made keeping in mind the above problems occurring in the prior art, and an object of the present invention is to provide an expandable interfusion cage wherein a spacer for expanding a branch part is structured to be moved in an easy manner when it is moved to expand the branch part, structural stability of the expanded branch part is improved, and the expansion of the branch part is increased.

An object of the invention is to provide an expandable interfusion cage by which stable movement of the spacer during expansion of the cage is obtained.

The object of the present invention has been achieved by an expandable interfusion cage having the features of claim 1.

### BRIEF DESCRIPTION OF THE DRAWINGS

The above and other objects, features and other advantages of the present invention will be more clearly understood from the following detailed description when taken in conjunction with the accompanying drawings, in which:

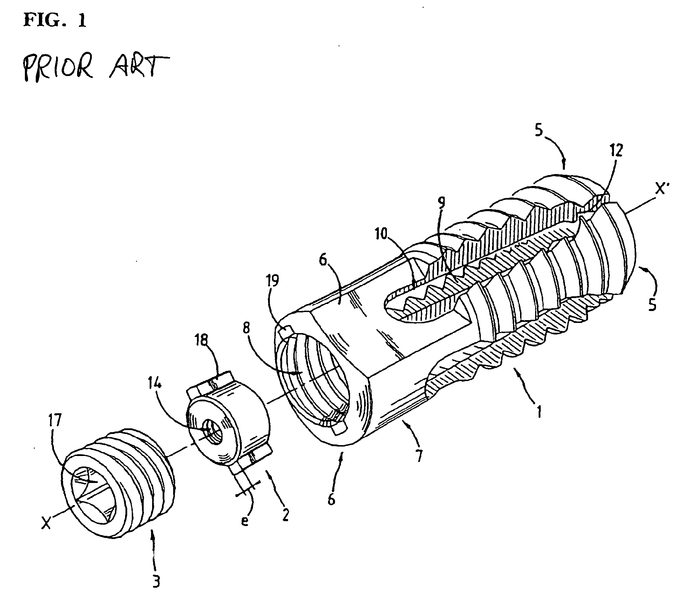

Fig. 1 is an exploded perspective view illustrating a conventional expandable interfusion cage;

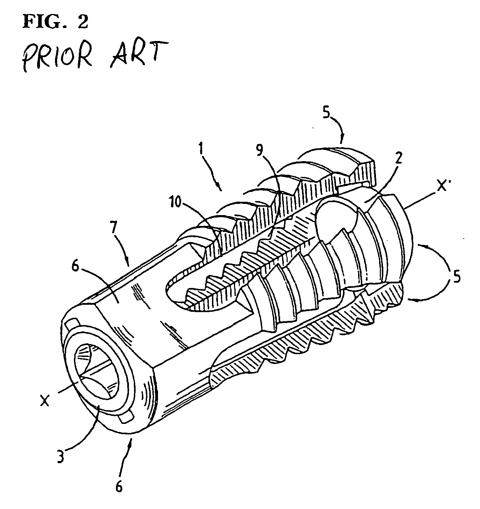

Fig. 2 is a perspective view illustrating an assembled state of the conventional expandable interfusion cage;
Fig. 3 is an exploded perspective view illustrating an expandable interfusion cage not forming part of the present invention;
Fig. 4 is a perspective view illustrating an assembled state of the expandable interfusion cage not forming part of the present invention;
Fig. 5 is a cross-sectional view taken along the line A-A of Fig. 4;
Fig. 6 is a cross-sectional view illustrating an operating state of the expandable interfusion cage not forming part of the present invention;
Fig. 7 is a perspective view illustrating an assembled state of a variation of the expandable interfusion cage not forming part of the present invention;
Figs. 8a through 8c are perspective views illustrating variations of a spacer used in the expandable interfusion cage not forming part of the present invention;
Fig. 9 is an exploded perspective view illustrating an expandable interfusion cage in accordance with the present invention; and
Fig. 10 is a perspective view illustrating an active position of the expandable interfusion cage according to the present invention.

### DETAILED DESCRIPTION OF THE INVENTION

Reference will now be made in greater detail to a preferred embodiment of the invention, an example of which is illustrated in the accompanying drawings. Wherever possible, the same reference numerals will be used throughout the drawings and the description to refer to the same or like parts.

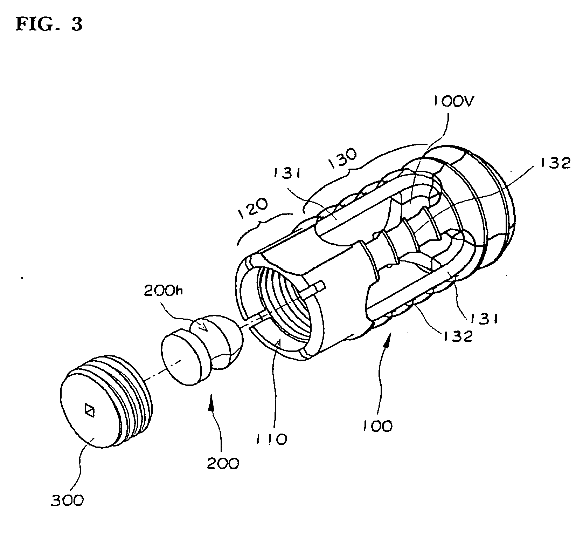

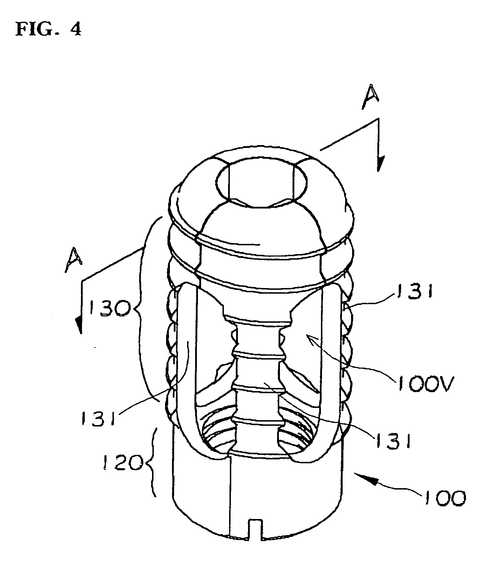

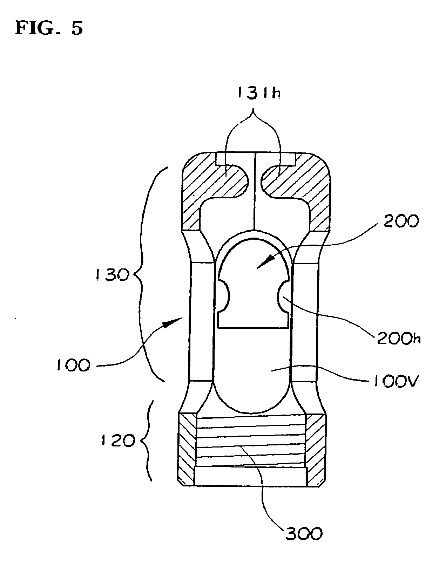

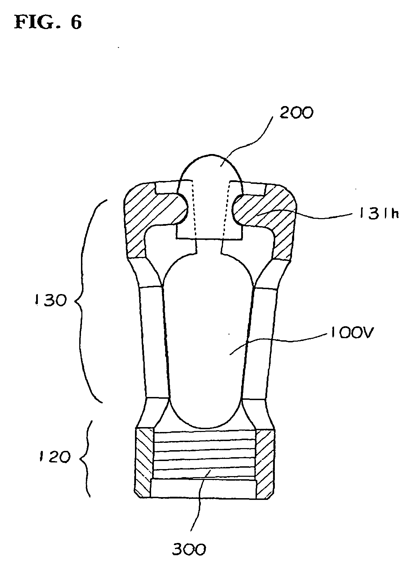

Fig. 3 is an exploded perspective view illustrating an expandable interfusion cage not forming part of the present invention; Fig. 4 is a perspective view illustrating an assembled state of the expandable interfusion cage not forming part of the present invention; Fig. 5 is a cross-sectional view taken along the line A-A of Fig. 4; and Fig. 6 is a cross-sectional view illustrating an operating state of the expandable interfusion cage not forming part of the present invention.

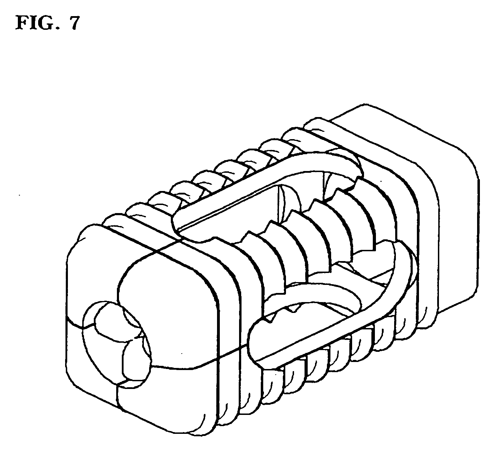

As shown in Figs. 3 through 6, an exemplary expandable interfusion cage comprises a cage body 100, a spacer 200 and a plug 300. The cage body 100 may be formed to have a quadrangular or cylindrical shape as shown in Figs. 7 or 3, respectively. The cage body 100 is composed of a seat part 120 and a branch part 130. As best shown in Fig. 3, the seat part 120 is pierced by an orifice 110 in which the plug 300 is threadedly assembled. A plurality of branches 131 constituting the branch part 130 are integrally formed at their proximal ends with the seat part 120.

When viewed as a whole, the cage body 100 constructed as mentioned above has a cylindrical shape, so that it defines therein an inside space 100v in which the spacer 200 is received and movably assembled.

Opposite outer surface portions of the seat part 120 constituting the cage body 100 are flattened, and a plurality of triangular valley portions are defined on upper and lower surfaces of the branches 131 of the branch part 130 to produce fixing portions 132. An opening is defined between two adjoining branches 131 constituting the branch part 130 so that the branches 131 can be expanded radially outward as the spacer 200 is moved in the inside space 100v.

Inward projections 131h are formed at the distal ends, respectively, of the branches 131 constituting the branch part 130 to project radially inward toward an axis of the cage body 100. Consequently, the inward projections 131h of the branches 131 cooperatively form an end of the cage body 100. That is to say, at the end of the cage body 100, which is opposite to the seat part 120, as shown in Fig. 5, the inward projections 131h of the branches 131 radially extend toward the axis of the cage body 100.

As can be readily seen from Figs. 3 and 5, the spacer 200 is inserted and movably assembled in the inside space 100v of the cage body 100. The spacer 200 has a head portion which possesses a bullet-shaped configuration and a base portion which possesses a disk-shaped configuration. The spacer 200 is defined with an annular groove 200h between the head portion and the base portion.

In the expandable interfusion cage, constructed as mentioned above, as shown in Fig. 6, as the spacer 200 is inserted into the inside space 100v of the cage body 100 and then moved toward the distal ends of the branches 131 of the branch part 130, the inward projections 131h formed at the distal ends of the branches 131 are engaged into the annular groove 200h of the spacer 200 to be fixedly maintained therein. At this time, as the inward projections 131h are biased radially outward by the spacer 200, the expandable interfusion cage of the present invention is expanded.

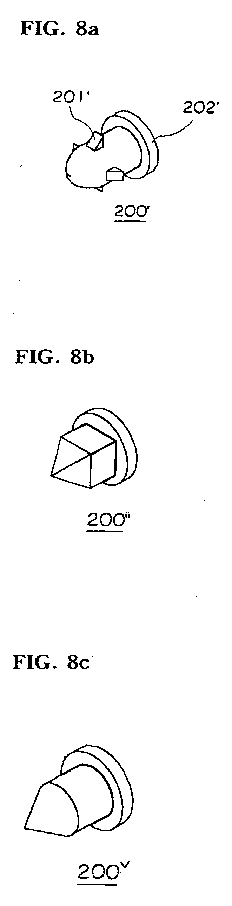

Figs. 8a through 8c are perspective views illustrating variations of a spacer used in the exemplary expandable interfusion cage. Referring to Fig. 8a, a spacer 200' has a head portion which possesses a bullet-shaped configuration and a base portion which is formed to serve as an engaging plate 202' possessing a diameter larger than that of the head portion. A plurality of engaging protuberances 201' each having a downwardly inclined face are formed on an outer surface of the spacer 200' directly below the head portion to be spaced apart one from another in a circumferential direction, in a manner such that the inward projections 131h formed at the distal ends of the respective branches 131 are engaged between the engaging plate 202' and the engaging protuberances 201' while expanding the cage body 100 radially outward. Fig. 8b illustrates a spacer 200" which has a quadrangular pyramid-shaped head portion, and Fig. 8c illustrates a spacer 200^{v} which has a conical head portion.

Although it was explained that the cage body has a shape of a hexahedron, a person skilled in the art will readily recognize that the cage body may have a cylindrical or a hexagonal shape while not departing from the scope of the present invention.

Also, while it was explained that the branch part 130 comprises four branches 131, a person skilled in the art will readily recognize that the number of branches is not limited to this and instead six or eight branches can be used to constitute the branch part.

After the expandable interfusion of the present invention is inserted and implanted in an intervertebral space of the spinal column of the human body, powdered bone fills the space between the vertebrae. A powdered-bone leakage prevention door may be provided to prevent the powdered bone from leaking between the vertebrae.

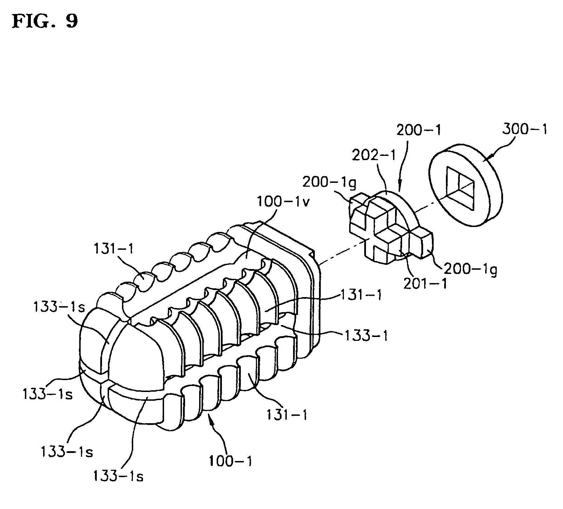

Fig. 9 is an exploded perspective view illustrating an expandable interfusion cage in accordance with the present invention.

As shown in Fig. 9, in an expandable interfusion cage in accordance with the present invention, as in the of the first embodiment, a cage body 100-1 defines therein an inside space 100-1v, and a branch part comprises four elongate branches 131-1. However, the cage body 100-1 of the invention is differentiated from the cage body 100 in that a guiding slit 133-1s communicated with an opening 133-1 is defined between two adjoining branches 131-1 when viewed on distal end surfaces of the branches 131-1 so that four guiding slits 133-1s cooperate to create a cross-shaped space.

In the present invention, a spacer 200-1 comprises a substantially cylindrical spacer body having a head portion 201-1 which is formed as a cross-shaped protrusion of a predetermined size to bias the branches 131-1 radially outward, and a base portion 202-1 which is formed as a disk possessing a diameter larger than that of the head portion 201-1 and has a pair of guide pieces 200-1g oppositely formed at both sides thereof, respectively.

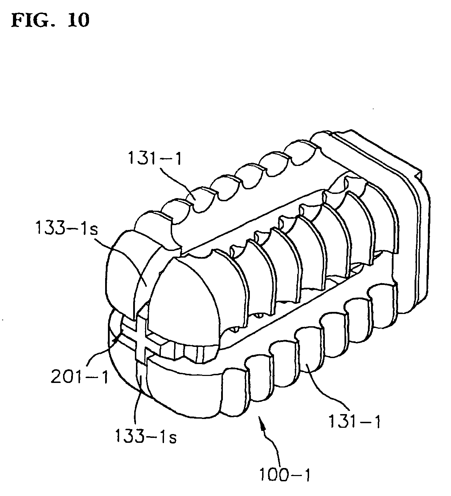

An expanded state of the cage according to the present invention, constructed as mentioned above, is illustrated in Fig. 10. As shown in Fig. 10, in the cage according to the present invention, the spacer 200-1 is movably assembled in the inside space 100-1v which is defined in the cage body 100-1. Then, as the guide pieces 200-1g are moved toward the distal ends of the branches 130-1 to be engaged in two guiding slits 133-1s when viewed at an end of the cage body 100-1 which is opposite to a seat part, the spacer 200-1 biases the branches 130-1 radially outward to allow the cross-shaped head portion 201-1 to expand the cage body 100-1.

Fig. 10 is a perspective view illustrating an active position of the expandable interfusion cage according to the present invention. As can be readily seen from the drawing, even though a substantial load is downwardly applied when the cage is inserted into a vertebra, an expanded state of the cage is stably maintained.

In the present invention, movement of the cage for biasing the inward projections radially outward to expand the cage body is more stably implemented, by which an expanded state of the cage body can be reliably maintained.

Although a preferred embodiment of the present invention has been described for illustrative purposes, those skilled in the art will appreciate that various modifications, additions and substitutions are possible, without departing from the scope of the invention as disclosed in the accompanying claims.

## Claims

1. An expandable interfusion cage comprising a cage body (100-1) of a quadrangular or cylindrical shape and a spacer (200-1), the cage body (100-1) including a seat part (120) which is pierced by an orifice and a branch part (130) which defines therein an inside space (100-1v) and has a plurality of elongate branches (101-1) integrally formed at their proximal ends with the seat part (120), with an opening (133-1) defined between two adjoining branches (101-1) to communicate with the inside space (100-1v), the spacer (200-1) being movably assembled in the inside space (100-1v) of the cage body (100-1) to expand the cage body radially outward, wherein the spacer (200-1) is engageable with the branches (101-1) while expanding the cage body (100-1),
wherein
a guiding slit (133-1s) communicated with the opening (133-1) is defined between two adjoining branches (131-1) when viewed on distal end surfaces of the branches (101-1) so that four guiding slits (133-1s) cooperate to create a cross-shaped space, and wherein the spacer (200-1) comprises a substantially cylindrical spacer body **characterized in that** the spaces body has a head portion which is formed as a cross-shaped protrusion of a predetermined size to bias the branches (131-1) radially outward, and a base portion (202-1) which is formed as a disk possessing a diameter larger than that of the head portion and has a pair of guide pieces (200-1g) oppositely formed at both sides thereof, respectively.

## Patentansprüche

1. Ein expandierbarer Zwischenwirbelfusionskäfig mit einem Käfiggehäuse (100-1) mit einer quaderförmigen oder zylindrischen Form und einem Distanzstück (200-1), wobei das Käfiggehäuse (100-1) einen Sitzteil (120) aufweist, welcher von einer Öffnung durchdrungen wird, und einen Astteil (130) aufweist, welcher einen Innenraum (100-1v) und eine Mehrzahl von langgestreckten Ästen (100-1) aufweist, welche einstückig an ihrem proximalen Ende an dem Sitzteil (120) angeformt sind, wobei eine Öffnung (133-1) zwischen zwei einander benachbarten Ästen (101-1) ausgebildet wird, um mit dem Innenraum (100-1v) in Verbindung zu stehen, wobei das Distanzstück (200-1) bewegbar in dem Innenraum (100-1v) des Käfiggehäuses (100-1) montiert ist, um das Käfiggehäuse radial nach außen zu expandieren, wobei das Distanzstück (200-1) mit den Ästen (101-1) in Eingriff steht, während das Käfiggehäuse (100-1) expandiert ist, wobei ein mit der Öffnung (133-1) in Verbindung stehender Führungsschlitz (133-1s) in Richtung der distalen Endfläche der Äste (101-1) betrachtet zwischen zwei benachbarten Ästen (131-1) ausgebildet ist, wobei vier Führungsschlitze (133-1s) so zusammenwirken, dass diese einen kreuzförmigen Raum ausbilden, und wobei das Distanzstück (200-1) einen im Wesentlichen zylindrischen Distanzstückkörper aufweist,
**dadurch gekennzeichnet, dass**
der Distanzstückkörper einen Kopfteil aufweist, welcher mit einem kreuzförmigen Vorsprung mit einer vorbestimmten Größe versehen ist, um die Äste (131-1) radial nach außen vorzuspannen, und einen Basisteil (202-1) aufweist, welcher als eine Scheibe ausgebildet ist, deren Außendurchmesser größer als derjenige des Kopfteils ist, und ein Paar von Führungsstücken (200-1g) aufweist, welche an jeweils gegenüberliegenden Seiten davon ausgebildet sind.

## Revendications

1. Une cage expansible d'inter liaison osseuse, comprenant un corps de cage (100-1) de forme quadrangulaire ou cylindrique et un organe d'espacement (200-1), le corps de cage (100-1) comportant une partie de siège (120) qui est traversée par un orifice et une partie de branche (130) qui définit à l'intérieur un espace intérieur (100-1v) et qui comporte une pluralité de branches allongées (101-1) formées monobloc à leurs extrémités proximales avec la partie de siège (120), avec une ouverture (133-1) définie entre deux branches contigües (101-1) pour communiquer avec l'espace intérieur (100-1v), l'organe d'espacement (200-1) étant assemblé de façon mobile dans l'espace intérieur (100-1v) du corps de cage (100-1) afin de dilater le corps de cage radialement vers l'extérieur, l'organe d'espacement (200-1) étant susceptible de venir en contact avec les branches (101-1) pendant qu'il dilate le corps de cage (100-1), dans laquelle cage expansible, une fente de guidage (133-1s) reliée à l'ouverture (133-1) étant définie entre deux branches contigües (131-1) lorsqu'on regarde sur les surfaces d'extrémité distales des branches (101-1), de sorte que quatre fentes de guidage (133-1s) coopèrent pour créer un espace en forme de croix et l'organe d'espacement (200-1) comprenant un corps sensiblement cylindrique de l'organe d'espacement, **caractérisé en ce que** le corps de l'organe d'espacement comporte une partie de tête qui est formée comme une saillie en forme de croix d'une taille prédéterminée pour solliciter les branches (131-1) radialement vers l'extérieur, et une partie de base (202-1) qui est formée comme un disque présentant un diamètre supérieur à celui de la partie de tête et qui comporte deux pièces de guidage (200-1g) formées respectivement de façon opposée sur deux faces de la partie de tête.
